# EUROPEAN PATENT APPLICATION

(11) **EP 4 119 101 A1**
(43) Date of publication of application: **18.01.2023**
(21) Application number: 21186250.3
(22) Date of filing: 16.07.2021
(51) Int. Cl.: A61F 2/44, A61F 2/46

(54) **EXPANDABLE AND ANGULARLY ADJUSTABLE SPINAL FUSION CAGES AND ASSOCIATED INSTRUMENTS**

(71) Applicant: Blue Ocean Spine GmbH, 78532 Tuttlingen (DE)
(72) Inventor: SALVERMOSER, Markus, 78532 Möhringen (DE); RICHTER, Jacob, 78532 Tuttlingen (DE); EISEN, Guntmar, 78532 Tuttlingen (DE)
(74) Representative: Stolmár & Partner Patentanwälte PartG mbB

(57) **Abstract**

The present disclosure provides adjustable spinal devices, instruments for implanting the spinal devices, methods for adjusting the height and lordosis angles of the spinal devices and methods for implanting such devices. An adjustable spinal fusion device includes an upper plate component having an outer surface for placement against an endplate of a vertebral body and a lower plate component having an outer surface for placement against an endplate of a vertebral body. The device further includes a first translation member configured to move longitudinally relative to the upper and lower plates to adjust a distance between the upper and lower plates and a second translation member configured to move longitudinally relative to the upper and lower plates to adjust an angle between the upper and lower plates.

## Description

### FIELD

The present disclosure relates to implantable devices for promoting fusion of adjacent bony structures and, more particularly, to implantable spinal fusion cages that can adjust in height and angle to address lordosis within an intervertebral space.

### BACKGROUND

Spinal fusion cages can be used to treat a variety of spinal disorders, including degenerative disc disease. The cages provide a space for inserting a bone graft material between adjacent vertebrae, and to promote fusion between the bones at this spine segment.

A problem common to traditional fusion cages is their lack of ability to maintain or restore the normal anatomy of the fused spine segment. In order to insert the cage between the adjacent vertebra, at least a portion, if not all, of the intervertebral disc is removed to make room for the cage. The removal of the entire disc or disc portion disrupts the normal lordotic or kyphotic curvature of the spine. Traditional fusion cages do not attempt to correct this curvature, and over time as the vertebrae settle around the implanted cages, kyphotic deformity results.

It is therefore desirable to provide spinal fusion cages that have the ability to maintain and restore the normal anatomy of the fused spine segment.

### SUMMARY

The present disclosure provides adjustable spinal devices and instruments for implanting the spinal devices. The present disclosure further provides methods for adjusting the height and lordosis angles of the spinal devices and methods for implanting such devices.

In one embodiment, an adjustable spinal fusion device includes an upper plate component having an outer surface for placement against an endplate of a vertebral body and a lower plate component having an outer surface for placement against an endplate of a vertebral body. The device further includes a first translation member configured to move longitudinally relative to the upper and lower plates to adjust a distance between the upper and lower plates (i.e., the height of the implant); and a second translation member configured to move longitudinally relative to the upper and lower plates to adjust an angle between the upper and lower plates (i.e., the angle of lordosis of the implant).

It is to be understood that both the foregoing general description and the following detailed description are exemplary and explanatory only and are not restrictive of the disclosure. Additional features of the disclosure will be set forth in part in the description which follows or may be learned by practice of the disclosure.

### BRIEF DESCRIPTION OF THE DRAWINGS

The accompanying drawings, which are incorporated in and constitute a part of this specification, illustrate several embodiments of the disclosure and together with the description, serve to explain the principles of the disclosure.
FIGS. 1-6 illustrate various views of an implantable spinal device according to the present disclosure;
FIGS. 7A-7C illustrate adjustment of the height and the angle of the implantable spinal device according to the present disclosure.
FIGS. 8-14 illustrate additional views of the implantable spinal device, including alternative embodiments;
FIGS. 15-17 illustrate various views of an instrument for inserting the implantable spinal device of the present disclosure between adjacent vertebral bodies in a patient; and
FIG. 18 illustrates the mechanism for angle adjustment of the spinal device of the present disclosure.

### DESCRIPTION OF THE EMBODIMENTS

This description and the accompanying drawings illustrate exemplary embodiments and should not be taken as limiting, with the claims defining the scope of the present disclosure, including equivalents. Various mechanical, compositional, structural, and operational changes may be made without departing from the scope of this description and the claims, including equivalents. In some instances, well-known structures and techniques have not been shown or described in detail so as not to obscure the disclosure. Like numbers in two or more figures represent the same or similar elements. Furthermore, elements and their associated aspects that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment. Moreover, the depictions herein are for illustrative purposes only and do not necessarily reflect the actual shape, size, or dimensions of the system or illustrated components.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

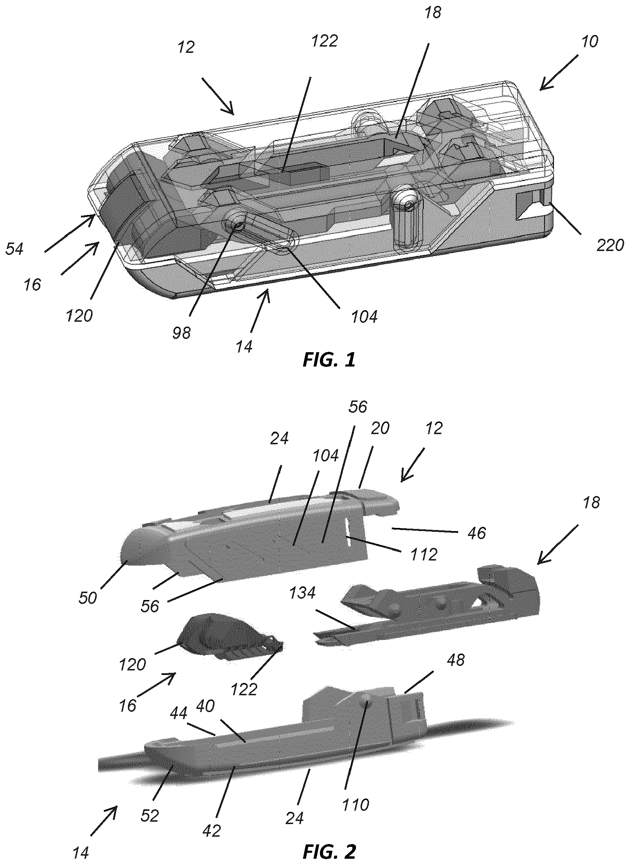

As shown in FIGS. 1-3, a spinal implant 10 according to the present disclosure is configured for placement between two vertebral bodies, preferably from a posterior approach outside of the facet joint (transforaminal lumbar interbody fusion or TLIF). The implant 10 includes upper and lower endplates 12, 14, a distal translation member 16 and a proximal translation member 18. Movement of the translation members 16, 18 in the longitudinal direction changes the height and angle of the endplates, as discussed in more detail below.

The spinal implant of the present disclosure has three different embodiments: (1) the base design; (2) the spring embodiment; and (3) the slot embodiment. The following description is common to all three embodiments. The additional features of the spring and slot embodiments will be described below.

The upper and lower endplates 12, 14 each include an outer surface 20, 22 for contacting the surface of a vertebral body. The outer surfaces 20, 22 are preferably roughened with a surface treatment that facilitates attachment to the vertebral body. The surface treatment preferably creates a diamond structure (e.g., diamond 20-1.5), although other patterns may be used. The upper and lower endplates also include central openings 24, 26 that extend through the entire endplates and, in one embodiment, are substantially aligned with each other. Similarly, the proximal translation member 18 includes a central opening or bore 28 that, in one embodiment, may be substantially aligned with the endplate openings 24, 26. These openings create space for the addition of bone graft or other substances into the implant, as well as to allow for bony ingrowth through the implant 10.

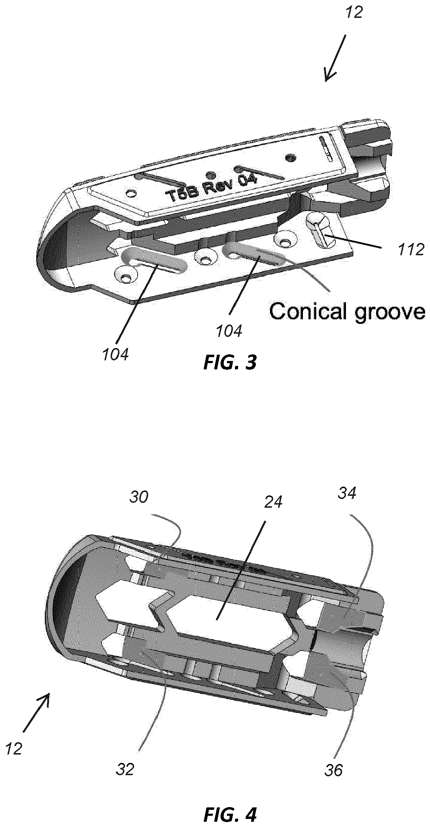

As shown in FIG. 4, the upper endplate 12 includes: (1) first and second distal sloped surfaces or ramps 30, 32 that are laterally spaced from each other near either side of the upper endplate and extend towards the lower plate 14 in the proximal direction; and (2) third and fourth proximal sloped surfaces or ramps 34, 36 that are laterally spaced from each other near either side of the endplate and also extend towards the lower endplate 14 in the proximal direction. These ramps interact with wedges on the proximal translation member to provide height adjustment of the implant (discussed below).

The lower endplate 14 includes a central channel 40 for receiving a lower portion 62 of the proximal translation member 18 therein, and for allowing longitudinal movement of the proximal translation member 18 relative to the endplates 12, 14. The central channel 40 is bound by first and second internal side walls 42, 44 that extend in a longitudinal direction from a proximal end to a distal end of the endplate.

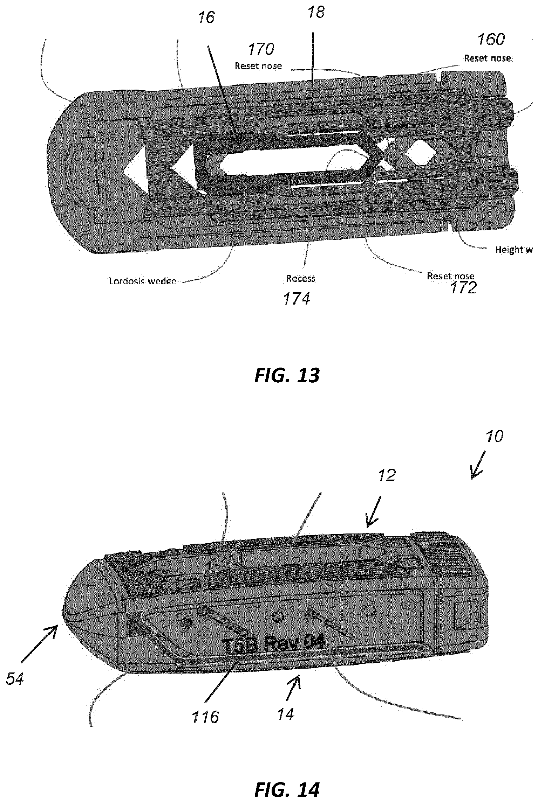

The upper and lower endplates have substantially open proximal ends 46, 48 and tapered distal ends 50, 52 to form a closed wedge-shaped nose 54 at the distal end of the implant (see FIG. 14). The upper endplate includes two side surfaces 56 that extend downward towards, and at least partially overlap with, the side surfaces 58 of the lower endplate.

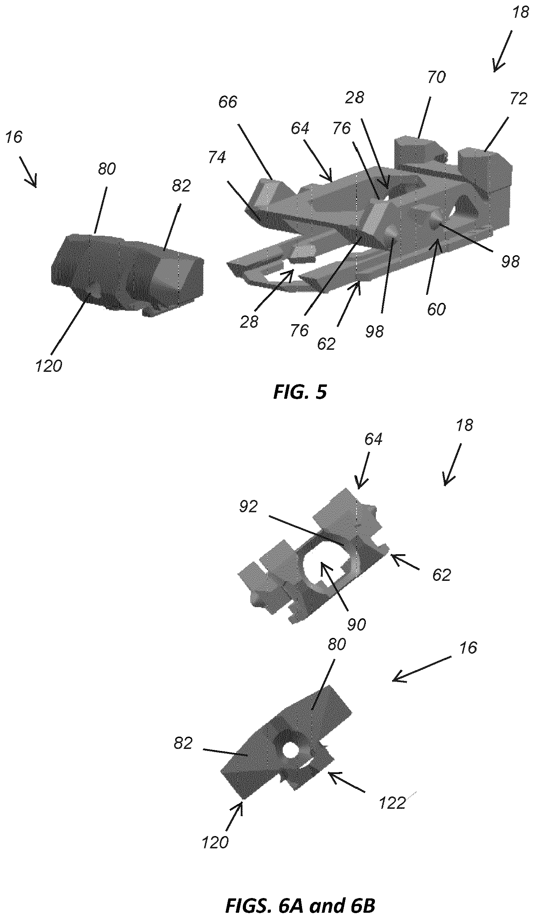

As shown in FIG. 5, the proximal translation member 18 includes a main body 60 that extends through a portion of the implant between the endplates. The main body 60 includes a lower portion 62 positioned within the channel 40 of the lower endplate 14 and configured to translate through the channel. The main body 60 further includes an upper portion 64 that is coupled to the lower portion 62 at the distal end of the translation member. The upper and lower portions 64, 62 both include a central opening 28 substantially aligned with the central openings 24, 26 of the endplates for receiving graft material and for allowing bony ingrowth.

The upper portion 64 of the proximal translation member 18 includes first and second distal sloped surfaces or wedges 66, 68 that are configured to contact and engage the first and second distal ramps 30, 32 of the upper endplate 12, and first and second proximal surfaces or wedges 70, 72 that are configured to contact and engage the first and second proximal ramps 34, 36 of the upper endplate 12. Longitudinal movement of the proximal translation member 18 causes these wedges to move along the ramps of the upper endplate, thereby moving the upper endplate towards or away from the lower endplate.

The upper and lower portions 62, 64 of the proximal translation member are separated from each other at their proximal ends to receive the distal translation member 16. Thus, a portion of the distal translation member extends into the proximal translation member. The upper portion 64 of the proximal translation member 18 further includes first and second sloped surfaces or ramps 74, 76 that are laterally spaced from each other and extend downwards towards the lower endplate 14 in the proximal direction. These ramps 74, 76 are configured to engage with wedges 80, 82 on the distal translation member 16 to adjust the angle of the implant, as discussed below.

As shown in FIG 6A, the proximal translation member 18 further includes a central bore 90 through at least its proximal end for receiving a first rod 202 of an insertion instrument 200 (discussed below). The bore 90 includes an annular mating feature configured to receive, and couple to, an enlarged distal end 206 of the rod 202. The mating feature includes a cavity or cut-out 92 sized to receive the enlarged distal end 206 of the rod 202. The cavity includes a proximal rim or projection that extends towards the longitudinal axis and has a smaller inner diameter than the inner diameter of the cavity 92 and the outer diameter of the enlarged distal end 206. The enlarged distal end 206 of the first rod 202 is tapered on its distal surface. The rim is sufficiently flexible to allow the tapered surface of the. enlarged distal end 206 to push past the rim (by bending it) and become secured within the cavity 92. The rim is sufficiently rigid to inhibit the enlarged end 206 of the rod 202 to push past it in the proximal direction when the rod is pulled in the proximal direction. Once the insertion instrument rod 202 is coupled to the proximal translation member 18, longitudinal translation of the rod 202 causes the proximal translation member 18 to move longitudinally relative to the endplates 12, 14, thereby moving the endplates towards or away from each other.

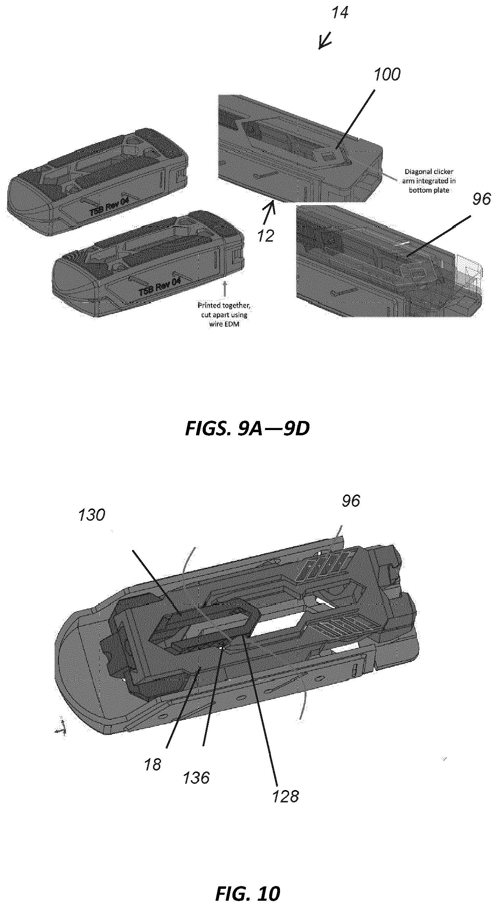

As shown in FIGS. 9 and 10, the lower endplate 14 further includes a substantially diagonal "clicker arm" 100 for providing discrete "steps" in the movement of the proximal translation member 18 relative to the endplates. These steps correlate with height adjustments of the endplates. To this end, the proximal translation member 18 comprises a series of projections or teeth 96 extending from its upper surface towards the upper endplate. The clicker arm 100 includes a plurality of projections (not shown) extending downwards and configured to extend into the spaces formed between the teeth 96 of the proximal translation member 18. As the proximal translation member 18 moves longitudinally, the projections are configured to move from the space between two teeth 96 to the space between adjoining teeth. These spaces provide the discrete steps or increments. In addition, the teeth 96 hold the translation member 18 in position relative to the endplate 12 to maintain a particular height or distance between the upper and lower endplates. The projections also inhibit reverse movement (i.e., distal movement) of the proximal translation member relative to the endplates.

Referring to FIG. 3, the proximal translation member 18 further includes one or more projections (i.e., conical pins 98) extending laterally away from the translation member 18. These conical pins 98 extend through slots 104 in the side surfaces 56 of the upper endplate and serve to stabilize the endplates during height and angular adjustment. In particular, the translation member includes at least two conical pins 98 on each side spaced longitudinally from each other and configured to ride through angled slots 104 in the side surfaces 56 of the upper endplate 12.

In the Slot embodiment (see FIGS. 2 and 3), the lower endplate 14 also includes a conical pin 110 extending laterally outward from each side of the endplate 14. These conical pins 110 are configured to ride through substantially vertical slots 112 in the sides of the upper endplate 12 to provide further positional stabilization.

In the Spring embodiment (see FIG. 14), the upper and lower endplates further include first and second flexible hinges or leaf springs 116 on either side of the endplates that couple the endplates together to further stabilize the endplates. The endplates are separate components that are only coupled to each other by the leaf springs 116 and the connections created by the various conical pins riding through slots in the upper endplate (discussed above). The leaf springs 116 are each formed as an elongate component that extends from a proximal portion of the lower endplate to a distal portion of the lower endplate. The elongate component has a relatively small cross-sectional area that creates flexibility in the elongate component as the endplates move relative to each other; allowing it to flex with this movement and remained coupled to the endplates. This provides positional stability to the endplates during height and angle adjustment.

As shown in FIGS. 5, 6, and 8, the distal translation member 16 has a main body 120 positioned near the distal end of the endplates and an extension member 122 extending proximally from the main body 120. The main body 120 further includes first and second sloped surfaces or wedges 80, 82 laterally spaced from each other and extending downwards towards the lower endplate 14 in the proximal direction. The wedges 80, 82 are configured to contact and engage the ramps 74, 76 on the proximal translation member 18 such that movement of the distal translation member 16 in the proximal direction relative to the proximal translation member 18 causes a distal end portion of the proximal translation member 18 to move upwards or away from the lower endplate 14. This movement causes the distal end portion of the upper endplate 12 to also move away from the lower endplate 14, thereby adjusting the angle between the endplates.

The extension member 122 of the distal translation member includes two longitudinal supports 128, 130 that form a central channel 132 therebetween. The longitudinal supports 128, 130 are positioned within a channel 134 formed in the lower portion 62 of the proximal translation member 18. The longitudinal supports 128, 130 are also configured to translate through this channel 134 during angle adjustment (discussed below).

Each of the longitudinal supports 128, 130 of the distal translation member 16 include a series of projections or teeth 136 formed on the outer side of these supports 128, 130. The teeth 136 are configured to engage two lateral projections 140, 142 extending from the inner surfaces of the lower portion 62 of the proximal translation member 18 to provide discrete "steps" as the distal translation member 16 is moved in relation to the proximal translation member 18. Each "step" corresponds to a specific angle change in the endplates and allows the endplates to be locked into a particular angle. The projections 140, 142 also inhibit reverse movement (i.e., distal movement) of the distal translation member 16 relative to the proximal translation member 18.

In the Slot and Spring embodiments, the distal translation member has a different overall shape. The extension member 122 is partially located beneath the main body 120 and partially extends from the main body 120 in the proximal direction.

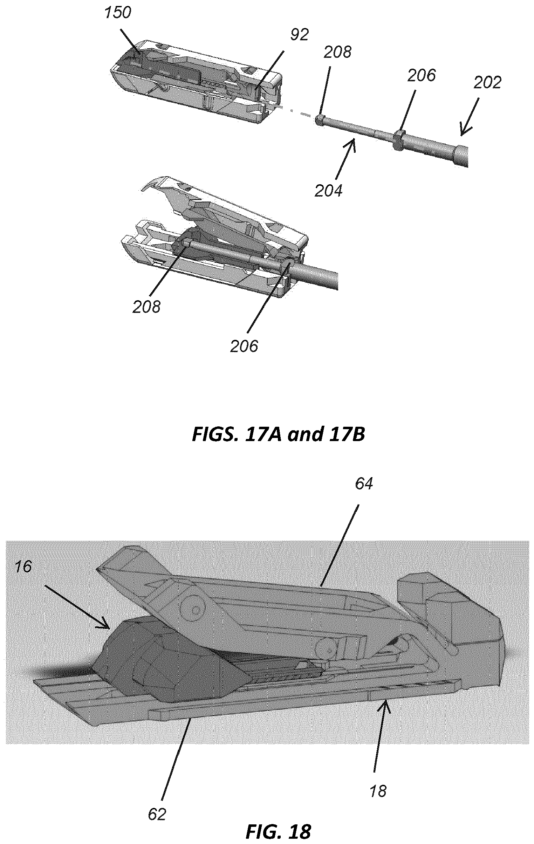

As shown in FIGS. 17A and 17B, the distal translation member 16 further includes a mating feature configured to receive, and couple to, an enlarged distal end 208 of a second rod 204 on the insertion instrument (discussed below). The mating feature includes a cavity or cut-out 150 sized to receive the enlarged distal end 208 of the rod 204. The cavity 150 includes a proximal rim or projection that extends towards the longitudinal axis and has a smaller inner diameter than the inner diameter of the cavity 150 and the outer diameter of the enlarged distal end 208. The enlarged distal end 208 of the rod 204 is tapered on its distal surface. The rim is sufficiently flexible to allow the tapered surface of the enlarged distal end 208 to push past the rim (by bending it) and become secured within the cavity 150. The rim is sufficient rigid to inhibit the enlarged end 208 of the rod 204 to push past it in the proximal direction when the rod 204 is pulled in the proximal direction. Thus, once the rod 204 is coupled to the distal translation member 16, longitudinal translation of the rod 204 causes the distal translation member 16 to move longitudinally relative to the proximal translation member 18 and the endplates 12, 14, thereby adjusting the angle of the endplates.

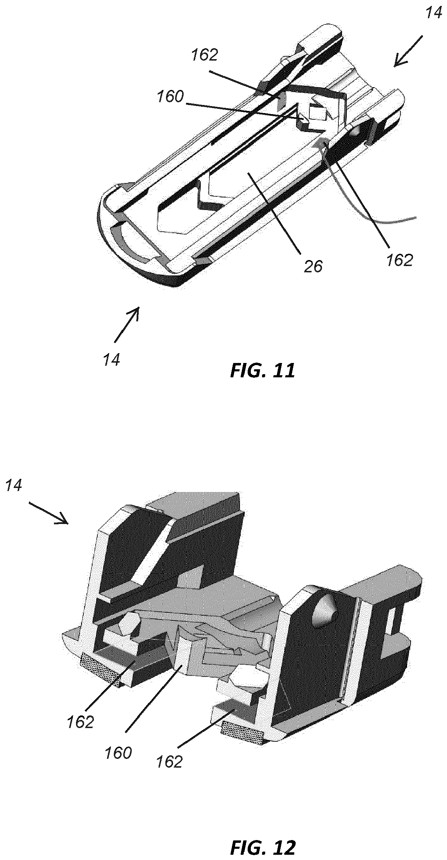

Referring now to FIGS. 11-13, the lower implant 14 can further comprise one or more reset noses 160, 170, 172, which are configured to release the latched latching components in a controlled manner if it is pushed. Thus, the height and the lordosis can be readjusted if they are adjusted above their specific target value. Pushing the reset nose 160 results in pushing the latching component, e.g., the latching nose 162, apart from the latching position. Thus, the component comprising the latching component can be readjusted. The reset nose 160 can be positioned near and attached to the flexible latching component.

As shown in FIG. 13, the proximal translation member 18 may include two reset noses 170, 172. If these noses 170, 172 are pushed, the distal translation member 16 can be readjusted. Nose 170, 172 can be pushed by rotating the locking rod 204 corresponding to the distal translation member 16 before it has reached the locking position, e.g., before entering the cage, and then by pushing the locking rod 204 into the implant until it has reached the reset noses 170, 172.

The lower implant 14 may also include a reset nose 160. If reset nose 160 is pushed, the proximal translation member 18 can be readjusted. It can be pushed by pushing the corresponding locking rod 202 into the implant beyond the locking position without rotating it. As shown, the reset nose 160 is preferably positioned on the lower endplate 14 and not on the upper endplate 12 because upper endplate 12 is lifted from the lower endplate 14. This would result in lifting the corresponding reset nose 160 away from the locking rod 202 which then cannot reach and push the reset nose 160. Preferably, the distal translation member comprises a recess 174 to enable the corresponding locking rod 202 to reach the reset position, especially if the distal translation member 16 is pulled to the most far position inside the proximal translation member 18.

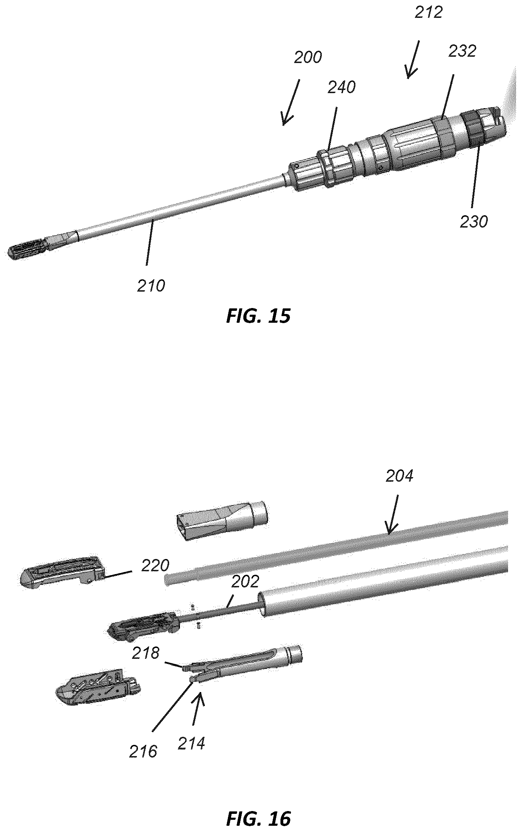

Referring now to FIGS. 15-17, the insertion instrument 200 comprises an elongated shaft 210 with a proximal handle 212 and a distal gripping element 214 for removable coupling to the implant. The distal gripping element 214 includes first and second gripping arms 216, 218 for coupling to proximal mating features 220 on either side of the upper endplate (a bayonet style connection). The distal gripping arms 216, 218 are coupled to an actuator 220 on the proximal handle 212 to move the arms 216, 218 in a substantially lateral direction relative to the longitudinal axis of the shaft 210. The arms 216, 218 can be moved together to hold the lower endplate 14 and moved apart to release the endplate 14.

The elongated shaft 210 includes inner and outer concentric rods 202, 204. The inner rod 202 can be extended from the handle 212 to the distal translation member 16 and the outer rod 204 can be extended from the handle 212 to the proximal translation member 18. The inner and outer rods 202, 204 are both attached to rotatable knobs 230, 232 on the proximal handle 212 for translating the rods 202, 204 longitudinally relative to the elongated shaft 210. The inner rod 202 has an enlarged distal end 206 that may be coupled to the cavity 150 of the distal translation member 16 and the outer rod 204 has an enlarged distal end 208 that can be coupled to the cavity 92 on the proximal translation member 18.

The handle 212 further includes a third rotatable knob 240 coupled to the shaft 210 for rotating the shaft 210 and the endplates therewith relative to the handle 212. This allows for rotation of the endplates without rotating the handle to facilitate ease of use during implantation.

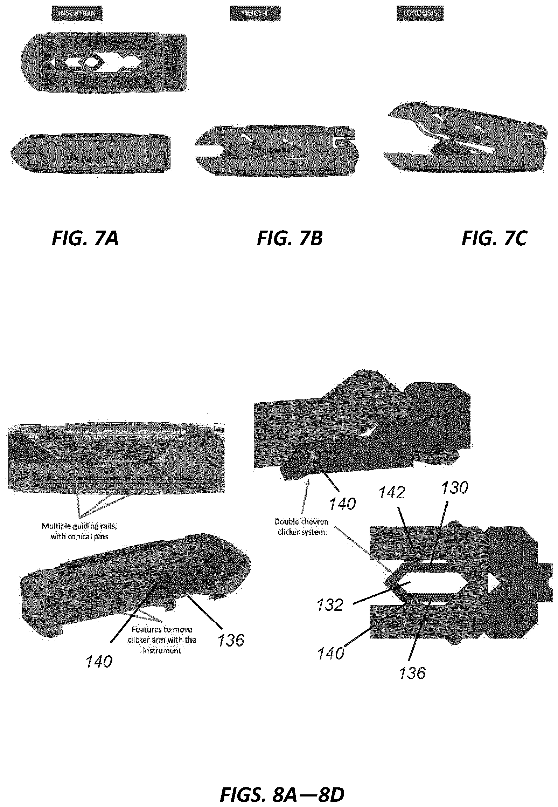

In use, the implant may be advanced into an intervertebral space in a collapsed configuration (see the resting state shown in FIG. 7A). To increase the height of the implant, the endplates are moved away from each other in a substantially parallel direction. To that end, inner and outer rods may be advanced distally and coupled to the translation members, as discussed above. The rotatable knob (green) on the handle may then be rotated to thereby withdraw the outer rod proximally. This translates both the proximal and distal translation members in the proximal direction. As the distal translation member moves in the proximal direction, its wedges engage with the upper endplate ramps such that the endplates move apart from each other in a substantially parallel direction (see expanded state shown in FIG. 7B).

To adjust the angle of the endplates, the rotatable knob (red) on the handle may be rotated, causing the inner rod to move in the proximal direction. The inner rod pulls the distal translation member proximal relative to both the endplates and the proximal translation member. The wedges on the distal translation member contact and engage the ramps on the proximal translation member, causing the distal end portion of the proximal translation member to move upwards away from the lower endplate. This also causes the distal end portion of the upper endplate to move away from the lower endplate. Since the proximal translation member does not move, the proximal ends of the endplates remain fixed relative to each other, thereby adjusting the angle of the upper endplate relative to the lower endplate (see angularly adjusted state shown in FIG. 7C).

The process of height and angle adjustment is reversible. The height and angle may also be adjusted independently of each other. For example, the above process can be reversed such that the inner rod is first withdrawn proximally to adjust angle, and then the outer rod is withdrawn proximally to adjust height.

The entire implant is fabricated through additive manufacturing techniques, such as 3D printing. The implant is formed layer by layer in the longitudinal direction from the proximal end to the distal end. Upon completion of manufacturing, the upper and lower endplates are substantially separated from each other except for their distal end portions. These portions are separated through wire EDM by cutting a substantially vertical line through these portions to form two separate components. In the Spring component, the endplates remain coupled together solely by the leaf spring. The endplates retain positional stability relative to each other during use by the conical knobs in the proximal translation member that slide through angled slots in the endplates, the conical knobs in the lower endplate that slide through the vertical slots in the upper endplate (Slot embodiment) and the leaf spring (Spring Embodiment).

In an exemplary embodiment, the implants are produced by Selective Laser Melting (SLM). For example, a substrate plate is fastened to an indexing table inside a chamber with a controlled atmosphere of inert gas (e.g., argon or nitrogen). Metal powder is applied flat to the substrate plate as a layer. The metal powder is preferably a titanium alloy, e.g. Ti-6AI-4V to enable biocompatibility. Each 2D slice of the cage is fused by selectively melting the metal powder via a laser. The laser has enough energy to fully melt or rather weld the metal particles to form solid metal. The substrate plate is lowered by the layer thickness (z-direction). New metal powder is applied and the process is repeated layer by layer until the part is complete. The completed part is removed from the substrate plate by cutting or breaking off.

Preferably, all components of the cage are printed nested within each other. Compared to separately 3D printing all components next to each other, a higher utilization rate can be achieved. This means that during 3D printing, a higher proportion which is melted and a lower proportion which stays as metal powder can be achieved. Thus, production time and costs can be reduced significantly.

After 3D printing, areas connecting single components of the cage are cut by electrical discharge machining (EDM) to enable their separate movement. Further, EDM can be used to realize smooth surfaces, e.g., to enable low-friction sliding of two components against each other. With EDM, the cage can also be removed from the substrate plate.

To lower production costs, several cages can be printed onto one substrate plate. In this case, before removing the cages, EDM can be used to simultaneously cut all cages placed on the substrate plate.

The implant may comprise one or more exhaust openings in the upper and lower endplates to allow for extraction of the metal powder remaining in the cage after 3D printing. Preferably, the exhaust opening is positioned on a lateral surface of the moving plate. It is also possible to position the exhaust opening on a horizontal surface of the cage, preferably on the base plate or on the moving plate. Preferably, the cage comprises multiple exhaust openings. Thus, more areas inside the cage are reachable and the metal powder can be extracted more efficiently. It is also possible to configure an external sliding mean, preferably a conical groove, in such a way that it can be additionally used as an exhaust opening. Therefore, the conical groove is deepened until a passage to the outside has been made.

Other embodiments will be apparent to those skilled in the art from consideration of the specification and practice of the embodiment disclosed herein. It is intended that the specification and examples be considered as exemplary only, with a true scope and spirit of the embodiment being indicated by the following claims.

## Claims

1. An adjustable spinal fusion device, comprising:
an upper plate component having an outer surface for placement against an endplate of a vertebral body;
a lower plate component having an outer surface for placement against an endplate of a vertebral body;
a first translation member configured to move longitudinally relative to the upper and lower plates to adjust a distance between the upper and lower plates; and
a second translation member configured to move longitudinally relative to the upper and lower plates to adjust an angle between the upper and lower plates.

2. The adjustable spinal fusion device of claim 1, further comprising an internal locking feature to prevent the upper plate from moving relative to the lower plate.

3. The adjustable spinal fusion device of claim 2, wherein the internal locking feature includes teeth for ratcheting.

4. The adjustable spinal fusion device of any preceding claim, wherein the first translation member comprises a mating feature for coupling to a shaft of an insertion instrument such that longitudinal movement of the shaft translates the first translation member in the longitudinal direction.

5. The adjustable spinal fusion device of any preceding claim, wherein the second translation member comprises a mating feature for coupling to a shaft of an insertion instrument such that longitudinal movement of the shaft translates the second translation member in the longitudinal direction.

6. The adjustable spinal fusion device of any preceding claim, wherein the first translation member includes a first movable wedge.

7. The adjustable spinal fusion device of claim 6, wherein the upper endplate comprises a ramp for cooperating with the first movable wedge.

8. The adjustable spinal fusion device of any preceding claim, wherein the second translation member includes a second movable wedge.

9. The adjustable spinal fusion device of claim 8, wherein the first translation member comprises a ramp for cooperating with the second movable wedge.

10. The adjustable spinal fusion device of any preceding claim, further being configured for use in the lumbar spine.
